# EUROPEAN PATENT SPECIFICATION

(11) **EP 0 981 349 B1**
(45) Date of publication and mention of the grant of the patent: **06.11.2002**
(21) Application number: 98918789.3
(22) Date of filing: 28.04.1998
(51) Int. Cl.: A61K 31/57, A61P 25/28, A61P 35/00

(54) **5-ALPHA-PREGNAN-3-BETA-OL-20-ONE SULFATE FOR THE TREATMENT OF TUMOURS AND CNS DISEASES**
5-ALPHA-PREGNAN-3-BETA-OL-20-ONE 3-SULFATE FÜR DI BEHANDLUNG VON TUMOREN UND ZNS KRANKHEITEN
5-ALPHA-PREGNAN-3-BETA-OL-20-ONE SULFATE POUR LE TRAITEMENT DES CANCERS ET DES MALADIES DU SNC

(30) Priority: 02.05.1997 US 850567
(43) Date of publication of application: 01.03.2000
(73) Proprietor: Wyeth, Madison, New Jersey 07940-0874 (US)
(72) Inventor: KAGAN, Michael, Z., Plainsboro, NJ 08536 (US); SHAH, Syed, Muzafar, East Hanover, NJ 07936 (US)
(74) Representative: Talbott, Dawn Jacqueline
(86) International application number: US9808456
(87) International publication number: WO98050042

(56) References cited:
- IRWIN R P ET AL: "STEROID POTENTIATION AND INHIBITION OF N-METHYL-D-ASPARTATE RECEPTOR-MEDIATED INTRACELLULAR CA++ RESPONSES: STRUCTURE- ACTIVITY STUDIES" JOURNAL OF PHARMACOLOGY AND EXPERIMENTAL THERAPEUTICS, vol. 271, no. 2, November 1994, pages 677-682, XP000609600
- MIJEONG PARK-CHUNG ET AL: "3ALPHA-HYDROXY-5BETA-PREGNAN-20-ONE SULFATE: A NEGATIVE MODULATOR OF THE NMDA-INDUCED CURRENT IN CULTURED NEURONS" MOLECULAR PHARMACOLOGY, vol. 46, no. 1, July 1994, pages 146-150, XP000609601
- TOWNSLEY, J.D.: "Further Studies on the Regulation of Human Placental Steroid 3-Sulfatase Activity" ENDOCRINOLOGY,1972, pages 172-181, XP002076176
- BAILLIE ET AL.: "SYNTHESIS OF SPECIFICALLY DEUTERIUM-LABELLED PREGNANOLONE AND PREGNANEDIOL SULPHATES FOR METABOLIC STUDIES IN HUMANS" STEROIDS, vol. 26, no. 4, 1975, pages 438-457, XP002076218
- MIJEONG PARK-CHUNG ET AL.: "Distinct Sites for Inverse Modulation of N-Methyl-D-Aspartate Receptors by Sulfated Steroids" MOL. PHARM., vol. 52, no. 6, 1997, pages 1113-1123, XP002076175
- EL-ETR ET AL.: "Opposing effects of different steroid sulfates on GABAA receptor-mediated chloride uptake" BRAIN RESEARCH, vol. 790, 1998, pages 334-338, XP002076177
- BAILLIE ET AL.: "PRODUCTION RATES AND METABOLISM OF SULPHATES OF 3beta-HYDROXY-5alpha-PREGNANE DERIVATIVES IN PREGNANT WOMEN" J. STEROID BIOCHEM., vol. 13, no. 12, 1980, pages 1473-1486, XP002076178
- BITRAN D ET AL: "ANXIOLYTIC EFFECT OF 3A-HYDROXY 5A BETA-PREGNAN-20-ONE:ENDOGENOUS METABOLITES OF PROGESTERONE THAT ARE ACTIVE AT THE GABAA RECEPTOR" BRAIN RESEARCH, vol. 561, no. 1, 4 October 1991, pages 157-161, XP002050235
- MICKAN ET AL.: "PREGNANOLONES AND PREGNENOLONE IN HUMAN MYOMETRIUM AT TERM OF PREGNANCY" J. STEROID BIOCHEM., vol. 11, no. 4, 1979, pages 1455-1459, XP002076219

## Description

The use of naturally occurring estrogenic compositions of substantial purity and low toxicity such as PREMARIN (conjugated equine estrogens) has become a preferred medical treatment for alleviating the symptoms of menopausal syndrome, osteoporosis/osteopenia in estrogen deficient women and in other hormone related disorders. The estrogenic components of the naturally occurring estrogenic compositions have been generally identified as sulfate esters of estrone, equilin, equilenin, 17-β-estradiol, dihydroequilenin and 17-β-dihydroequilenin (U.S. Patent 2,834,712). The estrogenic compositions are usually buffered or stabilized with alkali metal salts of organic or inorganic acids at a substantially neutral pH of about 6.5 to 7.5. Urea has also been used as a stabilizer (U.S. 3,608,077). The incorporation of antioxidants to stabilize synthetic conjugated estrogens and the failure of pH control with tris(hydroxymethyl)aminomethane (TRIS) to prevent hydrolysis is discussed in U.S. 4,154,820.

One of the compounds described herein, 5α-pregnan-3β-ol-20-one 3-sulfate ester sodium salt is a minor component of PREMARIN (conjugated equine estrogens), and is also commercially available.

Studies on the regulation of human placental steroid 3-sulfatase activity are reported by J D Townsley in Endocrinology, 1972 pages 172-181. T A Baillie et al describe the metabolism and production rates of 3β-hydroxy-5α-pregnan-20-one sulfate and the 3- and 3,20-sulfates of 5α-pregnan-3β,20α-diol in pregnant women in Steroids, vol. 26, no. 4, 1975 pages 438-457. Studies by R P Irwin et al showed that several pregn-5-ene steroids markedly potentiated N-methyl-D-aspartate mediated [Ca⁺⁺] responses, J. Pharm. Exp. Therap. vol. 271(2) 1994 pages 677-682. The ability of pregnenolone sulfate and epipregnanolone sulfate to compete with each other as well with other known classes of NMDA was examined by Mijeong Park-Chung et al in Mol. Pharm. vol. 52, no.6 1997 pages 1113-1123. El-Etr et al studied the opposing effects of different steroid sulfates on GABAₐ mediated chloride uptake in Brain Research vol. 790 1998 pages 334-338.

### DESCRIPTION OF THE INVENTION

In accordance with this invention, there is provided the use of a pharmaceutically acceptable salt of 5α-pregnan-3β-ol-20-one 3-sulfate ester in the preparation of a medicament for providing progestational therapy to a mammal. This invention also provides a pharmaceutically acceptance salt of 5α-pregnan-3β-ol-20-one 3-sulfate ester for use as a medicament.

Pharmaceutically acceptable salts of 5α-pregnan-3β-ol-20-one 3-sulfate ester include, but are not limited to, the alkali metal salts, alkaline earth metal salts, ammonium salts, alkylammonium salts containing 1-6 carbon atoms or dialkylammonium salts containing 1-6 carbon atoms in each alkyl group, trialkylammonium salts containing 1-6 carbon atoms in each alkyl group and tetraalkylammonium salts containing 1-6 carbon atoms in each alkyl group.

Alkali metal salts include sodium and potassium salts, particularly preferred are sodium salts. Alkaline earth metal salts include calcium and magnesium salts. Suitable alkyl groups include methyl, ethyl, propyl, butyl, pentyl and hexyl, preferred alkyl groups being methyl and ethyl. Where more than one alkyl group is present the groups may be the same or different. Preferred trialkylammonium salts are trimethylammonium salts and triethylammonium salts.

The present invention further provides compositions comprising pharmaceutically acceptable salt of 5α-pregnan-3β-ol-20-one 3-sulfate ester . In particular it provides compositions comprising at least 1% of pharmaceutically acceptable salt of 5α-pregnan-3β-ol-20-one 3-sulfate ester . One aspect of the present invention provides compositions wherein the only progestational agent is a pharmaceutically acceptable salt of 5α-pregnan-3β-ol-20-one 3-sulfate ester. Embodiments of the present invention include compositions wherein the only active compound is pharmaceutically acceptable salt of 5α-pregnan-3β-ol-20-one 3-sulfate ester. In these embodiments other excipients and carriers may be included but no further active materials are included.

The present invention also provides a process for the preparation of a pharmaceutically acceptable salt of 5α-pregnan-3β-ol-20-one 3-sulfate ester which comprises:
a) converting 5α-pregnan-3β-ol-20-one 3-sulfate ester to a pharmaceutically acceptable salt or
b) converting a pharmaceutically acceptable salt of 5α-pregnan-3β-ol-20-one 3-sulfate ester to a different pharmaceutically acceptable salt of 5α-pregnan-3β-ol-20-one 3-sulfate ester.

5α-pregnan-3β-ol-20-one 3-sulfate esters may be converted to pharmaceutically acceptable salts by neutralising the acid with an appropriate base, e.g. with an alkali metal carbonate, an alkaline earth metal carbonate or a primary, secondary, tertiary or quaternary amine carbonate. Alkali metal or alkaline earth metal salts may be prepared by using the appropriate alkali metal hydride e.g. sodium hydride, potassium hydride or lithium hydride.

A pharmaceutically acceptable salt of 5α-pregnan-3β-ol-20-one 3-sulfate ester may be converted to a different pharmaceutically acceptable salt of 5α-pregnan-3β-ol-20-one 3-sulfate ester by displacement, by using an ion exchange resin or by double decomposition (metastasis). Displacement of a weak base with a stronger one may be utilised to convert, e.g. an amine salt to an alkali metal salt or an alkaline earth metal salt using an appropriate base, e.g. a hydroxide. For example a trialkylamine salt such as a triethylamine salt may be converted to an alkali metal salt such as a sodium salt by treating it with an alkali metal hydroxide such as aqueous sodium hydroxide. The displacement may be carried out using an ion exchange resin. Alternatively one salt may be converted to another by double decomposition, e.g. an alkaline earth metal salt such as the calcium salt may be replaced with an alkali metal salt. E.g. the calcium salt of 5α-pregnan-3β-ol-20-one 3-sulfate ester may be dissolved in water followed by the addition of e.g. sodium carbonate. Insoluble calcium carbonate would then precipitate out to provide the sodium salt of 5α-pregnan-3β-ol-20-one 3-sulfate ester.

A pharmaceutically acceptable salt of 5α-pregnan-3β-ol-20-one 3-sulfate ester may be prepared by directly converting 5α-pregnan-3β-ol-20-one to a pharmaceutically acceptable salt of 5α-pregnan-3β-ol-20-one 3-sulfate ester. This may be performed by reacting it with the appropriate aminesulfurtrioxide complex, e.g. by reacting it with a trialkylaminesulfurtrioxide (such as triethylaminesulfurtrioxide complex) to provide the corresponding trialkylamine salt (such as the triethylamine salt). If desired the salt may then be converted to another salt of the invention as described above. Alkali metal or alkaline earth metal salts may be prepared by treating 5α-pregnan-3β-ol-20-one with the appropriate alkali metal hydride e.g. sodium hydride, potassium hydride or lithium hydride to produce the corresponding alkoxide *in situ* and then adding a trialkylaminesulfurtrioxide (such as triethylaminesulfurtrioxide complex) to provide the corresponding trialkylamine salt (such as the triethylamine salt). If desired the salt may then be converted to another salt of the invention as described above.

The present invention also provides pharmaceutically acceptable salts of 5α-pregnan-3β-ol-20-one 3-sulfate ester prepared by a chemical process, particularly those prepared according to the processes described above. The invention also provides pharmaceutically acceptable salts of 5α-pregnan-3β-ol-20-one 3-sulfate ester obtainable by such processes.

The compounds of this invention can be prepared from readily available starting materials using standard literature techniques or can be purchased, as with 5α-pregnan-3β-ol-20-one 3-sulfate ester sodium salt.

The progestational activity of a representative compound of this invention (5α-pregnan-3β-ol-20-one 3-sulfate ester sodium salt) was evaluated in an in vitro standard pharmacological test procedure. The procedure used and results obtained are briefly described below.

The results of these standard pharmacological test procedures demonstrate that the compounds of this invention are progestational. In this test procedure, the progestational activity of a compound is quantified based on its stimulation of alkaline phosphatase enzyme activity in T47D cells, a human breast cancer cell line which expresses high levels of progesterone receptors. This is a well established test procedure in which both the progestin receptors and the response stimulated by activated progestin receptors are endogenous to the cells. Cells are pre-conditioned in low serum medium for one day and then treated with test compounds. Alkaline phosphatase activity is measured 24 hr after treatment. Reference progestins, such as progesterone and medroxyprogesterone acetate, induce a 30 - 60 fold induction of alkaline phosphatase requiring only low nanomolar concentrations for activity. The alkaline phosphatase activity induced by progestins is blocked or inhibited by progestin receptor antagonists such as RU486 indicating the specificity of the response. When evaluated in this test procedure, 5α-pregnan-3β-ol-20-one 3-sulfate ester sodium salt, had an IC₅₀ of 5 x10⁻⁶ demonstrating progestational activity.

The neuroprotective and cognition enhancing effects of the compounds of this invention were evaluated in an in vitro standard pharmacological test procedure which measured the effects of 5α-pregnan-3β-ol-20-one 3-sulfate ester sodium salt, as a representative compound of this invention, on calcium and potassium channel currents. Briefly, the following procedure was used.

Whole cell recording techniques were used to record calcium and potassium currents from cultured hippocampal neurons. The compounds to be evaluated were made fresh each day in a 400 µM ethanol stock solution. The test compounds were diluted in saline to obtain a final concentration of 2 µM. The amplitude of calcium or potassium currents in control, test compound, and washout solutions was measured from at least 10 current traces for each condition. To compensate for rundown of the calcium current with time, control and washout currents were averaged. The current amplitude with drug was divided by the averaged control and washout current to determine the percent change. The means, standard deviations and errors for each test compound were calculated and significance from control was determined using the paired T-Test. 5α-pregnan-3β-ol-20-one 3-sulfate ester sodium salt enhanced increased potassium channel currents versus control by 21.76 ± 2.40 % (p = 0.0006), indicating that 5α-pregnan-3β-ol-20-one 3-sulfate ester sodium salt hyperpolarizes neurons, thereby allowing them to respond more readily to other stimuli. Calcium channel currents were significantly decreased (p = 0.044) from control decrease by 4.64 ± 1.64 % versus control) demonstrating the neuroprotective effects of 5α-pregnan-3β-ol-20-one 3-sulfate ester sodium salt. These results show that the compounds of this invention are useful as neuroprotective agents, in protecting against epileptic seizures, and in cognition enhancement.

The compounds of this invention are progestational agents. Based on the results obtained in the standard pharmacological test procedures, the compounds of the invention are useful as oral contraceptives (male and female), in hormone replacement therapy (particularly when combined with an estrogen), in the treatment of endometriosis luteal phase defects, benign breast and prostatic diseases and prostatic and endometrial cancers. The compounds of this invention are also useful in protecting against epileptic seizures, in cognition enhancement, in treating Alzheimer's disease, dementias, vasomotor symptoms related to menopause, and other central nervous system disorders The compounds of this invention are further useful in stimulating erythropoises.

The compounds of this invention can be used alone as a sole therapeutic agent or can be used in combination with other agents, such as other estrogens, progestins, or and androgens.

The compounds of this invention can be formulated neat or with a pharmaceutical carrier for administration, the proportion of which is determined by the solubility and chemical nature of the compound, chosen route of administration and standard pharmacological practice. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, lethicins, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellant.

Liquid pharmaceutical compositions which are sterile solutions or suspensions can be utilized by, for example, intramuscular, intraperitoneal or subcutaneous injection. Sterile solutions can also be administered intravenously. The compounds of this invention can also be administered orally either in liquid or solid composition form.

The compounds of this invention may be administered rectally or vaginally in the form of a conventional suppository. For administration by intranasal or intrabronchial inhalation or insufflation, the compounds of this invention may be formulated into an aqueous or partially aqueous solution, which can then be utilized in the form of an aerosol. The compounds of this invention may also be administered transdermally through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semipermiable membrane covering a reservoir containing the active ingredient with or without a carrier, or a matrix containing the active ingredient. Other occlusive devices are known in the literature.

The dosage requirements vary with the particular compositions employed, the route of administration, the severity of the symptoms presented and the particular subject being treated. Based on the results obtained in the standard pharmacological test procedures, projected daily dosages of active compound would be 0.02 µg/kg - 750 µg/kg. Treatment will generally be initiated with small dosages less than the optimum dose of the compound. Thereafter the dosage is increased until the optimum effect under the circumstances is reached; precise dosages for oral, parenteral, nasal, or intrabronchial administration will be determined by the administering physician based on experience with the individual subject treated. Preferably, the pharmaceutical composition is in unit dosage form, e.g. as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate number of any such compositions in package form.

## Claims

1. Use of a pharmaceutically acceptable salt of 5α-pregnan-3β-ol-20-one 3-sulfate ester in the preparation of a medicament for providing progestational therapy to a mammal.

2. Use according to claim 1, wherein the pharmaceutically acceptable salt of the 3-sulfate ester is an alkali metal salt, alkaline earth metal salt, ammonium salt, alkylammonium salts containing 1-6 carbon atoms or dialkylammonium salts containing 1-6 carbon atoms in each alkyl group, trialkylammonium salts containing 1-6 carbon atoms in each alkyl group and tetraalkylammonium salts containing 1-6 carbon atoms in each alkyl group.

3. Use of a pharmaceutically acceptable salt of 5α-pregnan-3β-ol-20-one 3-sulfate ester in the preparation of a medicament for treating or inhibiting cancers, central nervous system disorders, dementias, or Alzheimer's disease in a mammal.

4. Use according to claim 3, wherein the pharmaceutically acceptable salt of the 3-sulfate ester is an alkali metal salt, alkaline earth metal salt, ammonium salt, alkylammonium salts containing 1-6 carbon atoms or dialkylammonium salts containing 1-6 carbon atoms in each alkyl group, trialkylammonium salts containing 1-6 carbon atoms in each alkyl group and tetraalkylammonium salts containing 1-6 carbon atoms in each alkyl group.

5. Use of a composition of matter consisting essentially of a pharmaceutically acceptable salt of 5α-pregnan-3β-ol-20-one 3-sulfate ester in the preparation of a medicament for providing progestational therapy to a mammal.

6. Use according to claim 5, wherein the pharmaceutically acceptable salt of the 3-sulfate ester is an alkali metal salt, alkaline earth metal salt, ammonium salt, alkylammonium salts containing 1-6 carbon atoms or dialkylammonium salts containing 1-6 carbon atoms in each alkyl group, trialkylammonium salts containing 1-6 carbon atoms in each alkyl group and tetraalkylammonium salts containing 1-6 carbon atoms in each alkyl group.

7. Use of a composition of matter consisting essentially of a pharmaceutically acceptable salt of 5α-pregnan-3β-ol-20-one 3-sulfate ester in the preparation of a medicament for treating or inhibiting cancers, central nervous system disorders, dementias, or Alzheimer's disease in a mammal.

8. Use according to claim 7, wherein the pharmaceutically acceptable salt of the 3-sulfate ester is an alkali metal salt, alkaline earth metal salt, ammonium salt, alkylammonium salts containing 1-6 carbon atoms or dialkylammonium salts containing 1-6 carbon atoms in each alkyl group, trialkylammonium salts containing 1-6 carbon atoms in each alkyl group and tetraalkylammonium salts containing 1-6 carbon atoms in each alkyl group.

9. Use of a pharmaceutically acceptable salt of 5α-pregnan-3β-ol-20-one 3-sulfate ester in the preparation of a medicament providing cognition enhancement.

10. Use according to claim 9, wherein the pharmaceutically acceptable salt of the 3-sulfate ester is an alkali metal salt, alkaline earth metal salt, ammonium salt, alkylammonium salts containing 1-6 carbon atoms or dialkylammonium salts containing 1-6 carbon atoms in each alkyl group, trialkylammonium salts containing 1-6 carbon atoms in each alkyl group and tetraalkylammonium salts containing 1-6 carbon atoms in each alkyl group.

11. A pharmaceutically acceptable salt of 5α-pregnan-3β-ol-20-one 3-sulfate ester for use as a medicament.

12. A pharmaceutically acceptable salt of 5α-pregnan-3β-ol-20-one 3-sulfate ester for use as a medicament for:
a) providing progestational therapy,
b) treating or inhibiting cancers, central nervous system disorders, dementias, or Alzheimer's disease or
c) providing cognition enhancement
to a mammal in need thereof.

## Patentansprüche

1. Verwendung eines pharmazeutisch annehmbaren Salzes von 5α-Pregnan-3β-ol-20-on 3-Sulfatester bei der Herstellung eines Arzneimittels zum Vorsehen progestationaler Therapie an einem Säuger.

2. Verwendung gemäß Anspruch 1, wobei das pharmazeutisch annehmbare Salz des 3-Sulfatesters ein Alkalimetallsalz, Erdalkalimetallsalz, Ammoniumsalz, Alkylammoniumsalze, welche 1-6 Kohlenstoffatome enthalten, oder Dialkylammoniumsalze, welche 1-6 Kohlenstoffatome in jeder Alkylgruppe enthalten, Trialkylammoniumsalze, welche 1-6 Kohlenstoffatome in jeder Alkylgruppe enthalten, und Tetraalkylammoniumsalze, welche 1-6 Kohlenstoffatome in jeder Alkylgruppe enthalten, ist.

3. Verwendung eines pharmazeutisch annehmbaren Salzes von 5α-Pregnan-3β-ol-20-on 3-Sulfatester bei der Herstellung eines Arzneimittels zum Behandeln oder Hemmen von Krebsen, Störungen des zentralen Nervensystems, Demenz oder Alzheimer-Erkrankung in einem Säuger.

4. Verwendung gemäß Anspruch 3, wobei das pharmazeutisch annehmbare Salz des 3-Sulfatesters ein Alkalimetallsalz, Erdalkalimetallsalz, Ammoniumsalz, Alkylammoniumsalze, welche 1-6 Kohlenstoffatome enthalten, oder Dialkylammoniumsalze, welche 1-6 Kohlenstoffatome in jeder Alkylgruppe enthalten, Trialkylammoniumsalze, welche 1-6 Kohlenstoffatome in jeder Alkylgruppe enthalten, und Tetraalkylammoniumsalze, welche 1-6 Kohlenstoffatome in jeder Alkylgruppe enthalten, ist.

5. Verwendung einer chemischen Zusammensetzung, welche im Wesentlichen aus einem pharmazeutisch annehmbaren Salz von 5α-Pregnan-3β-ol-20-on 3-Sulfatester besteht, bei der Herstellung eines Arzneimittels zum Vorsehen progestationaler Therapie an einem Säuger.

6. Verwendung gemäß Anspruch 5, wobei das pharmazeutisch annehmbare Salz des 3-Sulfatesters ein Alkalimetallsalz, Erdalkalimetallsalz, Ammoniumsalz, Alkylammoniumsalze, welche 1-6 Kohlenstoffatome enthalten, oder Dialkylammoniumsalze, welche 1-6 Kohlenstoffatome in jeder Alkylgruppe enthalten, Trialkylammoniumsalze, welche 1-6 Kohlenstoffatome in jeder Alkylgruppe enthalten, und Tetraalkylammoniumsalze, welche 1-6 Kohlenstoffatome in jeder Alkylgruppe enthalten, ist.

7. Verwendung einer chemischen Zusammensetzung, welche im Wesentlichen aus einem pharmazeutisch annehmbaren Salz von 5α-Pregnan-3β-ol-20-on 3-Sulfatester besteht, bei der Herstellung eines Arzneimittels zum Behandeln oder Hemmen von Krebsen, Störungen des zentralen Nervensystems, Demenz oder Alzheimer-Erkrankung in einem Säuger.

8. Verwendung gemäß Anspruch 7, wobei das pharmazeutisch annehmbare Salz des 3-Sulfatesters ein Alkalimetallsalz, Erdalkalimetallsalz, Ammoniumsalz, Alkylammoniumsalze, welche 1-6 Kohlenstoffatome enthalten, oder Dialkylammoniumsalze, welche 1-6 Kohlenstoffatome in jeder Alkylgruppe enthalten, Trialkylammoniumsalze, welche 1-6 Kohlenstoffatome in jeder Alkylgruppe enthalten, und Tetraalkylammoniumsalze, welche 1-6 Kohlenstoffatome in jeder Alkylgruppe enthalten, ist.

9. Verwendung eines pharmazeutisch annehmbaren Salzes von 5α-Pregnan-3β-ol-20-on 3-Sulfatester bei der Herstellung eines Arzneimittels zum Vorsehen kognitiver Verstärkung.

10. Verwendung gemäß Anspruch 9, wobei das pharmazeutisch annehmbare Salz des 3-Sulfatesters ein Alkalimetallsalz, Erdalkalimetallsalz, Ammoniumsalz, Alkylammoniumsalze, welche 1-6 Kohlenstoffatome enthalten, oder Dialkylammoniumsalze, welche 1-6 Kohlenstoffatome in jeder Alkylgruppe enthalten, Trialkylammoniumsalze, welche 1-6 Kohlenstoffatome in jeder Alkylgruppe enthalten, und Tetraalkylammoniumsalze, welche 1-6 Kohlenstoffatome in jeder Alkylgruppe enthalten, ist.

11. Pharmazeutisch annehmbares Salz von 5α-Pregnan-3β-ol-20-on 3-Sulfatester zur Verwendung als Arzneimittel.

12. Pharmazeutisch annehmbares Salz von 5α-Pregnan-3β-ol-20-on 3-Sulfatester zur Verwendung als Arzneimittel zum:
a) Vorsehen progestationaler Therapie
b) Behandeln oder Hemmen von Krebsen, Störungen des zentralen Nervensystems, Demenz oder Alzheimererkrankung oder
c) Vorsehen kognitiver Verstärkung an einem Säuger, der dessen bedarf.

## Revendications

1. Utilisation d'un sel pharmaceutiquement acceptable d'un ester 3-sulfate de 5α-prégnan-3β-ol-20-one dans la préparation d'un médicament pour fournir une thérapie progestative à un mammifère.

2. Utilisation selon la revendication 1 dans laquelle le sel pharmaceutiquement acceptable de l'ester 3-sulfate est un sel de métal alcalin, un sel de métal alcalino-terreux, un sel d'ammonium, des sels d'alkylammonium contenant 1 à 6 atomes de carbone ou des sels de dialkylammonium contenant 1 à 6 atomes de carbone dans chaque groupement alkyle, des sels de trialkylammonium contenant 1 à 6 atomes de carbone dans chaque groupement alkyle et des sels de tétraalkylammonium contenant 1 à 6 atomes de carbone dans chaque groupement alkyle.

3. Utilisation d'un sel pharmaceutiquement acceptable d'un ester 3-sulfate de 5α-prégnan-3β-ol-20-one dans la préparation d'un médicament pour traiter ou inhiber des cancers, des troubles du système nerveux central, des démences ou la maladie d'Alzheimer chez un mammifère.

4. Utilisation selon la revendication 3, dans laquelle le sel pharmaceutiquement acceptable de l'ester 3-sulfate est un sel de métal alcalin, un sel de métal alcalino-terreux, un sel d'ammonium, des sels d'alkylammonium contenant 1 à 6 atomes de carbone ou des sels de dialkylammonium contenant 1 à 6 atomes de carbone dans chaque groupement alkyle, des sels de trialkylammonium contenant 1 à 6 atomes de carbone dans chaque groupement alkyle et des sels de tétraalkylammonium contenant 1 à 6 atomes de carbone dans chaque groupement alkyle.

5. Utilisation d'une composition de matière consistant essentiellement en un sel pharmaceutiquement acceptable d'un ester 3-sulfate de 5α-prégnan-3β-ol-20-one dans la préparation d'un médicament pour fournir une thérapie progestative à un mammifère.

6. Utilisation selon la revendication 5, dans laquelle le sel pharmaceutiquement acceptable de l'ester 3-sulfate est un sel de métal alcalin, un sel de métal alcalino-terreux, un sel d'ammonium, des sels d'alkylammonium contenant 1 à 6 atomes de carbone ou des sels de dialkylammonium contenant 1 à 6 atomes de carbone dans chaque groupement alkyle, des sels de trialkylammonium contenant 1 à 6 atomes de carbone dans chaque groupement alkyle et des sels de tétraalkylammonium contenant 1 à 6 atomes de carbone dans chaque groupement alkyle.

7. Utilisation d'une composition de matière consistant essentiellement en un sel pharmaceutiquement acceptable d'un ester 3-sulfate de 5α-prégnan-3β-ol-20-one dans la préparation d'un médicament pour traiter ou inhiber des cancers, des troubles du système nerveux central, des démences ou la maladie d'Alzheimer chez un mammifère.

8. Utilisation selon la revendication 7, dans laquelle le sel pharmaceutiquement acceptable de l'ester 3-sulfate est un sel de métal alcalin, un sel de métal alcalino-terreux, un sel d'ammonium, des sels d'alkylammonium contenant 1 à 6 atomes de carbone ou des sels de dialkylammonium contenant 1 à 6 atomes de carbone dans chaque groupement alkyle, des sels de trialkylammonium contenant 1 à 6 atomes de carbone dans chaque groupement alkyle et des sels de tétraalkylammonium contenant 1 à 6 atomes de carbone dans chaque groupement alkyle.

9. Utilisation d'un sel pharmaceutiquement acceptable d'un ester 3-sulfate de 5α-prégnan-3β-ol-20-one dans la préparation d'un médicament permettant une amélioration de la cognition.

10. Utilisation selon la revendication 9, dans laquelle le sel pharmaceutiquement acceptable de l'ester 3-sulfate est un sel de métal alcalin, un sel de métal alcalino-terreux, un sel d'ammonium, des sels d'alkylammonium contenant 1 à 6 atomes de carbone ou des sels de dialkylammonium contenant 1 à 6 atomes de carbone dans chaque groupement alkyle, des sels de trialkylammonium contenant 1 à 6 atomes de carbone dans chaque groupement alkyle et des sels de tétraalkylammonium contenant 1 à 6 atomes de carbone dans chaque groupement alkyle.

11. Sel pharmaceutiquement acceptable d'un ester 3-sulfate de 5α-prégnan-3β-ol-20-one pour une utilisation comme médicament.

12. Sel pharmaceutiquement acceptable d'un ester 3-sulfate de 5α-prégnan-3β-ol-20-one pour une utilisation comme médicament pour :
a) fournir une thérapie progestative,
b) traiter ou inhiber des cancers, des troubles du système nerveux central, des démences ou la maladie d'Alzheimer ou
c) permettre une amélioration de la cognition chez un mammifère qui en a besoin.
